(19) Europäisches Patentamt
European Patent Office
Office européen des brevets

(11) **EP 1 645 982 A2**

(12) **EUROPEAN PATENT APPLICATION**

(43) Date of publication:
**12.04.2006 Bulletin 2006/15**

(51) Int Cl.:
***G06F 19/00*** *(2006.01)*

(21) Application number: **05109342.5**

(22) Date of filing: **07.10.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **08.10.2004 US 617179 P**

(71) Applicant: **Draeger Medical Systems, Inc.**
**Danvers, MA 01923 (US)**

(72) Inventors:
• **Penny, Mark**
  **Newburyport, MA 01950 (US)**
• **Shaffer, Judith**
  **Easton, CT 06612 (US)**

(74) Representative: **Kietzmann, Lutz et al**
**Maiwald Patentanwalts GmbH**
**Neuer Zollhof 2**
**40221 Düsseldorf (DE)**

(54) **A system supporting acquisition and processing of user entered information**

(57) A system supporting acquisition and processing of user entered information includes a user interface processor for generating data representing at least one form composition display image. A user, using the form composition display image, is able to compose an electronic form comprising a set of questions to be answered by user data entry via at least one data entry display image. A form processor receives data, entered using the composed electronic form, concerning a particular entity and translates the received data into a value for use in supporting decision making for that particular entity.

Fig. 1

**Description**

**FIELD OF THE INVENTION**

The present invention relates to user data acquisition and processing systems and in particular to data acquisition and processing systems used for scoring of an entity and/or the status or condition of the entity.

**BACKGROUND OF THE INVENTION**

Screening techniques provide information for efficient early decision making. A fast method for determining a coarse initial estimate of the status or condition of an entity is to solicit answers to a set of predetermined questions, and to translate the answers to those questions into a value. Such a procedure is sometimes called scoring, and the value termed a score. Further testing and processing of the entity may be based on the initial score.

For example, in medical diagnosis and treatment, a predetermined set of medical questions, termed a score sheet, is used by a clinician. The clinician solicits and records answers to the questions from a particular patient and/or from other sources of data such as patient medical records, patient administrative records, laboratory tests, current medications as indicated by pharmacy records, patient monitoring and/or treatment devices, and so forth. From the answers to the set of questions in the score sheet, the clinician may calculate a value, termed a score, which is a coarse estimate of the patient condition.

Originally, such score sheets were implemented in paper form. This required manual action by the clinician to solicit and record answers from the patient; to locate, retrieve and evaluate relevant patient information from other data sources (i.e. administrative data, medical records, laboratory tests, pharmacy records, monitoring and/or treatment parameters, etc.); and to calculate the score. Electronic score sheets were developed to enable the clinician to interact with a processor to solicit and record answers to the questions. In addition, because the other relevant patient information data sources may be accessible by the processor, the clinician is able to access and evaluate those data sources and to manually type the answers into the electronic score sheet, without physically locating, retrieving and evaluating paper records. In addition, the processor is able to calculate the score from the answers provided by the clinician.

Whether in paper or electronic form, such score sheets are hard coded by developers. However, because the score sheets are hard coded, updates are burdensome, which often delays score sheet updates. For example, such score sheet updates may take months to be distributed from the developer location and to be implemented at a customer location. In addition, existing score sheet systems typically fail to provide a way for customers and/or users to customize a score sheet. That is, current score sheet systems are unable to adapt to the range of possible scores that may be needed by the different customers. For example, specific countries and/or hospital applications may require special unique scoring questions and/or score calculations. That is, customers and/or users may need to adapt their score sheets to accommodate new scoring calculation algorithms, regulatory requirements and/or updates to scoring algorithms in a time efficient manner. A system is desired which addresses these deficiencies and related problems.

**BRIEF SUMMARY OF THE INVENTION**

In accordance with principles of the present invention, a system supporting acquisition and processing of user entered information includes a user interface processor for generating data representing at least one form composition display image. A user, using the form composition display image, is able to compose an electronic form comprising a set of questions to be answered by user data entry via at least one data entry display image. A form processor receives data, entered using the composed electronic form, concerning a particular entity and translates the received data into a value for use in supporting decision making for that particular entity.

**BRIEF DESCRIPTION OF THE DRAWING**

In the drawing:

Fig. 1    is a block diagram of a system according to principles of the present invention;

Fig. 2    is a more detailed block diagram of a portion of the system illustrated in Fig. 1 according to invention principles;

Fig. 3    is a block diagram of a processing system on which the system illustrated in Fig. 1 and Fig. 2 may be implemented according to invention principles;

Fig. 4    is an image of a graphical user interface which may be used to gather user entered information in the system illustrated in Fig. 1 and Fig. 2 according to invention principles;

Fig. 5    is an image of a graphical user interface which may be used by the system illustrated in Fig. 1 and Fig. 2 to generate data representing the user data entry image illustrated in Fig. 4 according to invention principles; and

Fig. 6    is an image of a graphical user interface which may be used by the system illustrated in Fig. 1 and Fig. 2 to generate data representing a score calculation based on user data entry according to invention principles.

**DETAILED DESCRIPTION OF THE INVENTION**

As used herein, a processor operates under the control of an executable application to (a) receive information from an input information device, (b) process the information by manipulating, analyzing, modifying, converting and/or transmitting the information, and/or (c) route the information to an output information device. A processor may use, or comprise the capabilities of, a controller or microprocessor, for example. The processor may operate with a display processor or generator. A display processor or generator is a known element for generating signals representing display images or portions thereof. A processor and a display processor comprise any combination of, hardware, firmware, and/or software.

An executable application as used herein comprises code or machine readable instructions for conditioning the processor to implement predetermined functions, such as those of an operating system, information acquisition system or other information processing system, for example, in response user command or input. An executable procedure is a segment of code or machine readable instruction, sub-routine, or other distinct section of code or portion of an executable application for performing one or more particular processes. These processes may include receiving input data and/or parameters, performing operations on received input data and/or performing functions in response to received input parameters, and providing resulting output data and/or parameters.

A user interface (UI), as used herein, comprises one or more display images, generated by the display processor under the control of the processor. The UI also includes an executable procedure or executable application. The executable procedure or executable application conditions the display processor to generate signals representing the UI display images. These signals are supplied to a display device which displays the image for viewing by the user. The executable procedure or executable application further receives signals from user input devices, such as a keyboard, mouse, light pen, touch screen or any other means allowing a user to provide data to the processor. The processor, under control of the executable procedure or executable application manipulates the UI display images in response to the signals received from the input devices. In this way, the user interacts with the display image using the input devices, enabling user interaction with the processor or other device.

A form, as used herein, is a type of UI display image. A form UI display image includes display elements, such as textual display, which prompt the user to enter particular information; and display elements, such as text boxes, check boxes etc., into which the user, using the input devices, may enter the particular information.

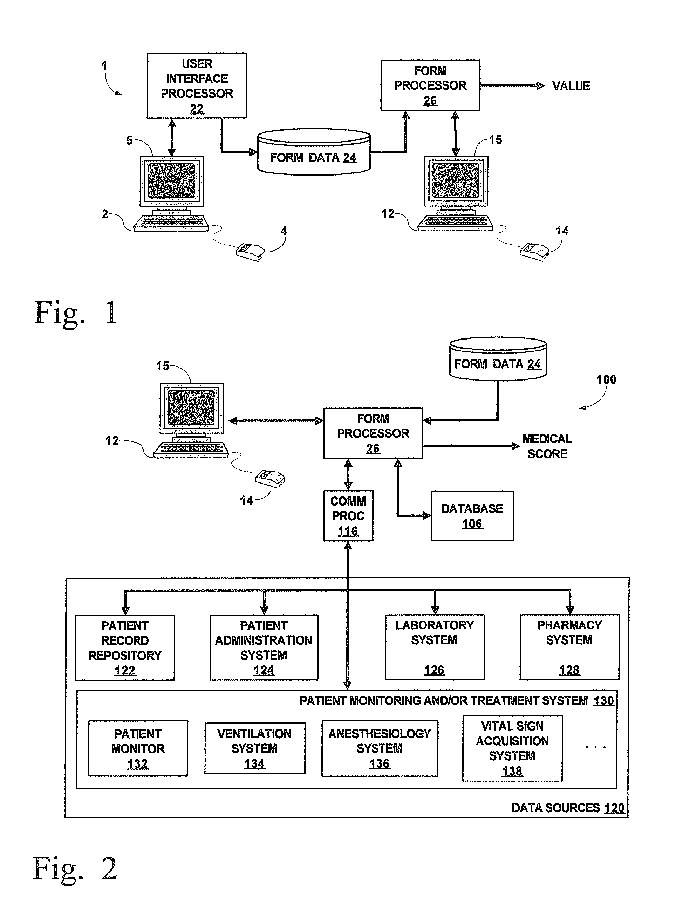

Fig. 1 is a block diagram of a system 1 according to principles of the present invention. In Fig. 1, a bidirectional terminal of a user interface processor 22 is coupled to a corresponding terminal of a first user interface device consisting of a keyboard 2, a mouse 4 and a monitor 5. An output terminal of the user interface processor 22 is coupled to an input terminal of a form data repository 24. An output terminal of the form data repository 24 is coupled to an input terminal of a form processor 26. A bidirectional terminal of the form processor 26 is coupled to a corresponding terminal of a second user interface device consisting of a keyboard 12, a mouse 14 and a monitor 15. An output terminal of the form processor 26 generates a signal representing a value.

In operation, the user interface processor 22 interacts with a user termed a form composer via the first user interface device (2, 4, 5) to compose an electronic form. The electronic form consists of a set of questions. The user interface processor 22 generates data representing at least one form composition display image and provides this data to the monitor 5 in the first user interface device (2, 4, 5) which displays the form composition display image. The form composer interacts, via the first user interface device (2, 4, 5), with the form composition display image to compose individual questions in the set of questions. In response, the user interface processor 22 produces data representing the composed electronic form including at least one data entry display image. The data entry display image or images display the set of questions in the electronic form. This electronic form representative data is stored in the form data repository 24.

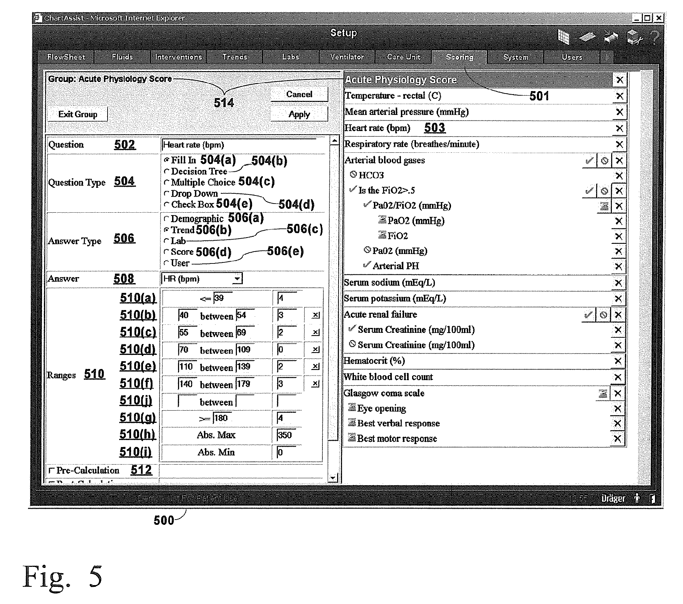

The operation of the user interface processor 22 may be better understood by reference to Fig. 5. Fig. 5 is an illustration of a form composition graphical user interface (GUI) 500 which may be used by the system illustrated in Fig. 1 to generate data representing at least a portion of a data entry display image. In the illustrated embodiment, the form composition GUI 500 is a part of a larger system, and is represented by a tab 501 labeled "Scoring". When the user activates the scoring tab 501, the GUI 500 of Fig. 5 is displayed.

The GUI 500 supports creation of an individual question of the set of questions. The right hand side of the GUI 500 lists the questions which are currently composed. Additional questions may be added and questions may be deleted. When a question is highlighted on the right hand side of the GUI 500, details about that question are displayed on the left hand side of the GUI 500 and those details may be edited by the form composer. In the illustrated embodiment, the "Heart rate (bpm)" question 503 is highlighted on the right hand side of the GUI 500. Thus, details about that question 503 are illustrated on the left hand side of the GUI 500.

Referring to the left hand side of the GUI 500, a section 502, labeled "Question" allows a form designer to designate a label or title or description of the question being composed by the GUI 500. In the illustrated embodiment, the question label is "Heart rate (bpm)". This is the label displayed in the list of questions on the right hand side of the GUI 500.

A section 504, labeled "Question Type" allows the form composer to select from among a plurality of predetermined question types. In the illustrated embodiment, the form designer may, using the illustrated radio buttons, select one of five predetermined questions types, although one skilled in the art understands that other question types may also be allowed. A first type of question 504(a) is "Fill In". This type is represented in the electronic form by a text box. The answer to a fill in question may be a textual value or a numeric value. The fill in question may include pre-calculations, i.e. calculations automatically made without user interaction based on the answers to sibling questions of this question; or post-calculations, i.e. calculations automatically made without user interaction after the answer has been filled in by the user. Fill in questions may also be of a pop-up type which means they initiate a pop-up box displaying a series of other questions. The answers to the pop-up questions may be processed to provide the answer to the fill in question.

A second type of question 504(b) is a "Decision Tree" question. This question may be represented by a'yes/no' multiple choice question or by a check box. A decision tree question is answered by the user and controls the display of corresponding other subsets of questions, possibly including more decision tree questions. That is, a 'yes' answer will cause the subsequent display of a first subset of questions while a 'no' answer will cause the subsequent display of a second subset of questions.

A third type of question 504(c) is a "Multiple Choice" question. This question may be represented by a set of radio buttons. The user may select one of a predetermined plurality of answers as designated by the form composer. A fourth type of question 504(d) is a "Drop Down" question. This question is similar to a multiple choice question except that it is represented by an option or drop down list containing the predetermined plurality of answers. The user may select one of the answers designated by the form composer. A fifth type of question 504(e) is a "Check Box" question. A user may either select or unselect a check box, thus providing a 'yes/no', 'true/false', 'present/not present', etc., indication.

The form composer may specify that the answer to the question being composed be automatically retrieved from one of a plurality of data sources. A section 506, labeled "Answer Type" permits the form composer to specify a data source containing the answer to the question being composed. The form composer may select, using appropriate radio buttons, a data source to be associated with this question. In the illustrated embodiment, there are five different data sources: patient demographics 506(a), trends 506(b), laboratory results 506(c), scores 506(d), and user data entry 506(e). One skilled in the art understands that other data sources may be present and may be selected by the form composer to be associated with the question being composed.

A section 508, labeled "Answer" permits the form composer to specify an identifier for the answer to this question. The identifier may be used when specifying subsequent calculations, such as pre-calculations for subsequent questions, post-calculations for this question or subsequent questions, calculation of the score value, and so forth. In the illustrated embodiment, the answer identifier is "HR (bpm)".

The sections described above (502, 504, 506, 508) are generally applicable to all questions. The sections which follow solicit information from the form composer necessary to compose a question of the type specified by the question type entry in section 504. In the illustrated embodiment, the selected question type is 'fill-in' and the expected answer data is numeric. For numeric 'fill-in' questions, answer data is partitioned into ranges and the respective ranges are assigned corresponding score values. A section 510 in the GUI 500 allows the form composer to specify the ranges and to assign respective numerical score values to the ranges. A first range 510(a) consists of heart rates below 39 beats per minute (bpm) and is assigned a score value of 4; a second range 510(b) consists of heart rates between 40 and 54 bpm and is assigned a score value of 3; a third range 510(c) consists of heart rates between 55 and 69 bpm and is assigned a score value of 2; a fourth range 510(d) consists of heart rates between 70 and 109 bpm and is assigned a score value of 0; a fifth range 510(e) consists of heart rates between 110 and 139 bpm and is assigned a score value of 2; a sixth range 510(f) consists of heart rates between 140 and 179 bpm and is assigned a score value of 3; and a seventh range 510(g) consists of heart rates above 180 bpm and is assigned a score value of 4. An absolute maximum heart rate of 350 bpm is set in entry 510(h) and an absolute minimum heart rate of 0 bpm is set in entry 510(i). The form composer may edit the ranges and score values in section 510 and may add further ranges by filling in the entry 510(j) or delete a range by activating the "x" button for that range.

A section 512 may be checked to specify pre-calculations, as described above. Further sections (not shown but accessible using the scroll bar) are available for the form designer to designate other options and aspects of the question being composed, such as, for example, post-calculations.

The user interface processor 22 (Fig. 1) generates data representing form composition display images containing different sections soliciting other information from the form composer for other question types. For example, for 'multiple choice' and 'drop down' type questions, a list of the acceptable answers, and score values associated with the respective answers in the list is specified; and for a check box, a score value may be associated with a checked answer and a different score value associated with an unchecked answer.

The questions may also be evaluated for roll up into a group. A group is a collection of questions the answers to which, in combination, may be used to calculate a group score value. Groups are "rolled up" by answering the grouped questions. The rolled up group score value may be: the summation of the score values for the answers to the questions in a group, the highest score value in the group, or some other combination of the score values for the answers to the questions in the group. In the illustrated embodiment, the "Heart rate (bpm)" question is part of the "Acute Physiology Score" group, as indicated by the label 514. The questions in this group are listed on the right hand side of the GUI 500, also indicated by the label 514.

More specifically, in the illustrated embodiment, each question in the group is associated with a score value. When the question "Heart rate (bpm)" is specified in a calculation, the score value for that question is substituted into the equation used to perform that calculation. That is, if the patient has a heart rate of 62, the score value for the "Heart rate (bpm)" question is 2. The score value 2, representing the heart rate, is used in the equation for calculating the group score. The group value, therefore, is a numeric value related to the respective score values for the questions in the group. The group score value, in turn, may be combined with other group score values, and/or with score values of other questions to form an overall score value. In this case, the group score value is represented by the name of the group. In Fig. 5. the name of the group is "Acute Physiological Score" 514.

A master equation may be composed to calculate an overall score value. This equation can perform mathematical functions as well as apply basic mathematical operands. The master equation may access score values from the answers to individual questions and/or from the rolled up score from the answers to groups of questions.

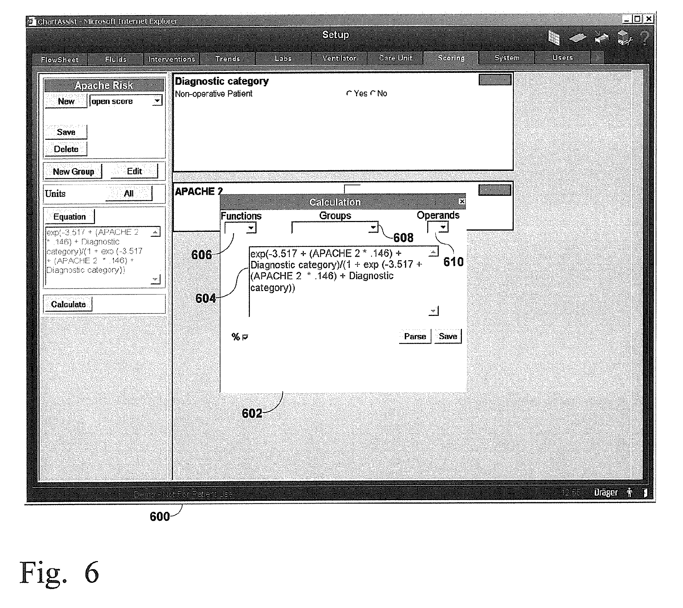

Fig. 6 is an image of a graphical user interface 600 which allows a form composer to compose a master equation to generate data representing the overall score value calculation based on user data entry. A dialog box 602 is displayed by the user interface processor 22 (Fig. 1) in which the form composer may compose the master equation. A text box 604 allows the form composer to type in the equation. A drop down list box 606 permits the form composer to access a list of supported mathematical functions and to automatically insert them into the equation being composed in the text box 604. A drop down list box 608 permits the form composer to access a list of question groups and to automatically insert an identifier representing a selected group score value into the equation being composed in the text box 604. A drop down list box 610 permits the form composer to access a list of supported mathematical operands and to automatically insert them into the equation being composed. In addition, numerical constants may be entered manually by the form composer. Equation (1), below, is the mathematical equation for the score value represented by the entry in dialog box 602 of Fig. 6, where "APACHE 2" represents the group score value for a group of questions labeled "APACHE 2" (Fig. 5 - 514) and "Diagnostic category" is the answer label (Fig. 5 - 508) representing the score for the question associated with that answer.

$$SCORE = \frac{e^{-3.517+0.146 \cdot APACHE\,2 \cdot + Diagnostic\,category}}{1 + e^{-3.517+0.146 \cdot APACHE\,2 + Diagnostic\,category}} \tag{1}$$

As described above, data representing the electronic form, based on the entries made by the form composer in response to the form composition display images, such as those illustrated in Fig. 5 and Fig. 6, is stored in the data repository 24.

Referring again to Fig. 1, the form processor 26 retrieves the electronic form representative data from the form data repository 24 and interacts, via the second user interface device (12, 14, 15), with a user to receive data representing answers to the questions in the electronic form. At least one data entry display image in the electronic form is displayed on the monitor 15 of the second user interface device (12, 14, 15). The user provides answers concerning a particular entity via the second user interface device (12, 14, 15). The form processor 26 translates the answer data received from the user into a value, as described in more detail below. This value acts as a score to support decision making for the particular entity.

The respective questions composed by the form composer using the user interface processor 22 are represented by data in the form data repository 24. A portion of this data specifies a data entry image element enabling a data entry user to enter the required data. The form processor 26 reads the data and generates an electronic form graphical user interface containing the data entry image elements corresponding to the data in the form data repository 24.

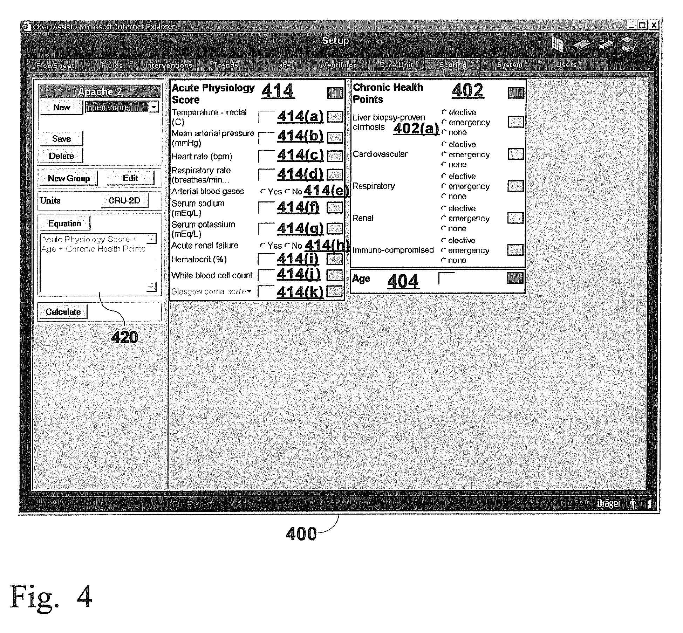

The operation of the form processor 26 may be better understood by reference to Fig. 4, Fig. 5 and Fig. 6. Fig. 4 is an image of a graphical user interface 400 which may be used to gather user entered data in the system illustrated in Fig. 1. Fig. 4 illustrates an electronic form with data entry image elements displaying two groups of questions and one ungrouped question. The first group of questions is the "Acute Physiology Score" group 414, the second group of questions is a "Chronic Health Points" group 402, and the ungrouped question 404 is labeled "Age". The data entry group "Acute Physiology Score" 414 display element corresponds to the "Acute Physiology Score" form composition group 514 illustrated in Fig. 5, and will be described in more detail below. The "Chronic Health Points' group 402 and the ungrouped question "Age" 404 are composed and displayed in a similar manner.

The list of questions on the right hand side of the GUI 500 in Fig. 5 are represented by data in the form data repository 24. The form processor 26 retrieves this data and generates the data entry elements in the Acute Physiology Score group 414 in the data entry GUI 400 in Fig. 4. The question "Temperature - rectal (C)" is the first question listed on the right hand side of the form composition GUI 500 and corresponds to the first question 414(a) displayed on the data entry display element 414 in the data entry GUI 400. The question "Mean arterial pressure (mmHg)" is the second question listed on the right hand side of the GUI 500 and corresponds to the second question 414(b) displayed on the data entry display element 414 of GUI 400, and so forth. The questions, 414(a), 414(b), 414(c), 414(d), 414(f), 414(g), 414(i) and 414(j), are fill in type questions expecting numerical data. A data entry user enters the numeric data (i.e. rectal temperature in degrees Celsius for question 414(a)) in the data box associated with the question.

The fifth question 414(e) is a decision tree question labeled "Arterial blood gases" and is represented by a multiple choice display element having two choices: "Yes' and "No". If "yes" is chosen, then a display element (not shown) asking the series of questions listed on the right hand side of the GUI 500 (Fig. 5) in the "Arterial blood gases" section is displayed, otherwise it is not displayed. Similarly, the question 414(h) is a decision tree question labeled "Acute renal failure" and is represented by a multiple choice display element having two choices: "Yes' and "No". If "yes" is chosen, then a display element (not shown) asking the series of questions listed on the right hand side of the GUI 500 in the "Acute renal failure" section is displayed, otherwise it is not displayed. The question 414(k) is a drop down type, as indicated by the small triangle to the right of the question label "Glasgow coma score". When the data entry user activates the triangle, a list of acceptable values drops down, and the user may select the appropriate value, which is then displayed in the text box associated with the question 414(k).

Referring to group 402 "Chronic Health Points", a first question 402(a), labeled "Liver biopsy-proven cirrhosis" is a multiple choice question. The multiple choice display element is represented by a set of radio buttons representing permitted answers: "elective", "emergency", and "none". A data entry user selects the radio button representing the appropriate answer to the question. The remaining questions in the "Chronic Health Points" group 402 are also multiple choice questions.

One skilled in the art will understand that there is no limit to the number of groups and/or ungrouped questions which may be placed in an electronic form. One skilled in the art will also understand that multiple GUI display images, similar to the GUI 400 illustrated in Fig. 4, may be required to display questions and receive answers.

When the respective questions in a group have been answered, the answer score values are determined and are rolled up into a group score value. The group score values and answer score values for ungrouped questions are processed by a corresponding composite score valuing equation to calculate a composite score value. In Fig. 4, the composite score value equation is illustrated in text box 420, and is the sum of the "Acute Physiology Score", the "Age", and the "Chronic Health Points" score values. This score, in turn, is designated as the "Apache 2" score value. The "Apache 2" score value is used in Fig. 6 in the master equation to generate the overall score value, as described above.

Referring again to Fig. 1, one skilled in the art understands that the first user interface device (2, 4, 5) and the second user interface device (12, 14, 15) may be the same physical keyboard (2, 12), mouse (4, 14) and monitor (5, 15). One skilled in the art also understands that the first user, interacting with the first user interface device (2, 4, 5) and composing the electronic form, may be a different user than the second user, interacting with the second user interface device (12, 14, 15) providing answers concerning a particular entity. For example, in a medical information context, the first user may be e.g. a clinical system administrator composing or modifying the composition of an electronic medical score sheet. The second user may be e.g. a clinician generating a medical score representing the status or condition of a particular patient. In this case, the value produced by the form processor 26 is a numerical value, representing a medical score, and is used to support clinical decision making for the particular patient.

As described above, some of the answers to the questions composed by the form composer may be found in data sources. Fig. 2 is a more detailed block diagram of a portion 200 of the system 1 illustrated in Fig. 1 in a medical clinical environment including elements necessary to access such data sources. In Fig. 2, those elements which are the same as those illustrated in Fig. 1 are designated by the same reference numbers and are not described in detail below. Fig. 2 illustrates the form data repository 24, form processor 26 and second user interface device (12, 14, 15).

In Fig. 2, further respective bidirectional terminals of the form processor are coupled to corresponding terminals of a database 106, and a communications processor 116. A second bidirectional terminal of the communications processor 106 is coupled to respective corresponding terminals of a set of data sources 120. The data sources include a patient record repository 122, patient administration system 124, a laboratory system 126, a pharmacy system 128 and a patient monitoring and/or treatment system 130. The patient monitoring and/or treatment system 130 includes a patient monitor 132, a ventilator system 134, an anesthesiology system 136, a vital sign acquisition system 138 and any other such patient monitoring and/or treatment device which may communicate with the communications processor 116.

In operation, the form processor 26 displays the display image or images associated with the electronic form, and interacts with a user via the second user interface device (12, 14, 15) to receive data representing answers to the questions in the electronic form, as described above. If the form composer has specified, e.g. in section 506 (Fig. 5), that the answer is preexisting in a data source, then the form processor 26 automatically accesses the specified data source, in a manner to be described in more detail below, to retrieve the answer, and place the answer in the appropriate display element in the data entry display image. In this manner, a user does not need to manually find, access, interpret, and re-enter preexisting data into the electronic score sheet form.

When a form composer associates a question with one of the data sources 120, data representing this association is stored in the database 106. This data includes the question, the data source associated with the data source, one or more communications protocols which may be used to access the data source, one or more formats in which the data in the data source may be stored and/or communicated. The database 106 may also store any other information necessary to access the data in the data sources 120, retrieve that data and insert it into the answer data entry elements in the electronic score sheet form automatically.

When the form processor 26 conditions the monitor 15 to display a question in a data entry display element, the form processor 26 accesses the database 106 to determine if that question is associated with a data source. If so, the form processor 26 conditions the communications processor 116 to bidirectionally communicate with the associated one (122, 124, 126, 128, 130, 132, 134, 136, 138, etc.) of the data sources 120 to identify the data in the data source which represents the answer to the associated question. The identified data is retrieved and sent to the form processor 26 via the communications processor 116 by that data source (122, 124, 126, 128, 130, 132, 134, 136, 138). The form processor 26 places that data in the answer data entry element associated with that question. The data entry user may then answer the remaining unanswered questions in the electronic score sheet form. The score value is then calculated based on the data entered by the user and the data automatically acquired from the data sources.

The data relating to the communications protocols and data formats allows the communications processor 116 to communicate with data sources 120 having any communications protocol and data format. By maintaining this data, different data sources 120 using different communications protocols and different data formats, manufactured by different manufacturers, may be properly accessed by the form processor 26 via the communications processor 116.

The transfer of the data from the data sources 120 into the electronic score sheet form is performed automatically. It may be initiated automatically with no user interaction, or may be initiated in response to a user command to process the composed form. In response to the user command, all associated data sources are accessed and the retrieved data placed in the electronic score sheet form.

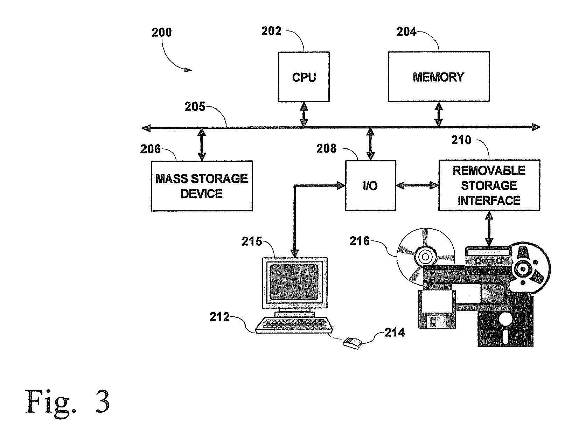

Fig. 3 is a block diagram of a processing system 200 on which the system illustrated in Fig. 1 and Fig. 2 may be implemented. The processing system 200 includes a central processing unit (CPU) 202, a memory 204, a mass storage device 206, and an input/output interface 208 coupled together by a computer bus 205. The input/output (I/O) interface 208 is coupled to a user interface consisting of a monitor 215, a keyboard 212 and a pointing device, which in the illustrated embodiment is a mouse 214. The I/O interface 208 is also coupled to a removable storage interface 210 capable of retrieving data from or storing data on one or more electronic data storage media 216. The electronic data storage media 216 may include magnetic devices such as reel-to-reel computer tape, cassette tapes, and magnetic disk media such as floppy disks and so forth. The electronic data storage media 216 may also include optical devices, such as digital video disk (DVD) or compact disk (CD) and so forth. One skilled in the art understands that any such electronic data storage media 216 may be used, such as portable storage devices including semiconductor memory integrated circuits. The I/O interface 208 may also be coupled to other peripheral devices (not shown) such as printers or communications devices for communicating with remote systems, local area networks (LANs) or wide area networks (WANs) such as the internet.

In operation, the CPU 202 operates as a processor which executes the machine readable instructions forming an executable application and/or executable procedures. Those machine readable instructions are stored in the memory 204, which may consist of read-only memory (ROM) and/or read/write memory (RAM). The CPU 202 retrieves the machine readable instructions from the memory 204 and executes them to perform the operations of the information acquisition system, as described above.

In the illustrated embodiment, the I/O processor 208 includes a display processor which, in response to commands from the CPU 202, generates signals representing the GUI display images described above and illustrated in Fig. 4 to Fig. 6, and supplies those image representative signals to the monitor 215 which displays the images. The I/O processor 208 also receives user commands and data from the keyboard 212 and/or mouse 214 and provides that information to the CPU 202. The CPU 202 responds to the received user commands and data to control the operation of the information acquisition system as described above.

Data may be retrieved from and stored in the mass storage device 206. For example, the mass storage device 206 may provide storage for the form data repository 24 (Fig. 1 and Fig. 2). The mass storage device 206 may also store data representing the machine readable instructions forming the executable application and/or executable procedures. The CPU 202 may retrieve the executable application and/or executable procedures from the mass storage device 206 and store them in the memory 204. The CPU 202 may then retrieve the machine readable instructions from the memory 204 and execute the executable application and/or executable procedures to perform the information acquisition activities described above.

Data may also be retrieved from and stored in electronic data storage media 216 via the removable storage interface 210. Any data may be stored in and/or retrieved from the electronic data storage media. More specifically, in the illustrated embodiment, the machine readable instructions in the executable application and/or executable procedures forming the information acquisition system may be stored in a electronic data storage medium. The CPU 202 may condition the I/O processor 208 to retrieve the executable application and/or executable procedures from the appropriate electronic data storage medium via the removable storage interface 210, and to store the executable application and/or executable procedures in the mass storage device 206 and/or the memory 204. The CPU 202 may then execute the executable application and/or executable procedures in the memory 204 to perform the information acquisition activities described above.

## Claims

1. A system supporting acquisition and processing of user entered information, comprising:

   a user interface processor for generating data representing at least one form composition display image enabling a user to compose an electronic form comprising a set of questions to be answered by user data entry via at least one data entry display image; and
   a form processor for receiving data entered using said composed electronic form concerning a particular entity and translating received data into a value for use in supporting decision making for said particular entity.

2. A system according to claim 1, wherein said at least one form composition display image, enabling a user to compose said electronic form, supports user creation of an individual question of said set of questions by selection from a plurality of predetermined question types.

3. A system according to claim 2, wherein said at least one form composition display image supports user creation of an individual question by enabling a user to select at least one data entry image element from a plurality of different data entry image elements enabling at least two of: (a) text data entry, (b) numerical value entry, (c) a multiple choice question, (d) an option list and (e) a check box, for display in said at least one data entry display image in said composed form.

4. A system according to claim 1, wherein said at least one form composition display image supports user entry of a calculable expression for translating data received in response to said set of questions in said composed form into said value.

5. A system according to claim 1, further comprising a database for associating individual questions of said set of questions with one or more data sources for providing data comprising answers to said associated questions.

6. A system according to claim 5, further comprising a communication processor for using said database to automatically bidirectionally communicate with said one or more data sources to acquire data representing answers to said associated questions.

7. A system according to claim 5, further comprising a communication processor for using said database to automatically bidirectionally communicate with said one or more data sources to acquire data representing answers to said associated questions in response to a user command to process said composed form.

8. A system according to claim 5, wherein said database associates said data sources with at least one of: (a) communication protocols and (b) data formats for use in bidirectionally communicating with said data sources to acquire data representing answers to said associated questions.

9. A system according to claim 5, wherein said form processor uses

said user data entered in response to said set of questions in said composed form, and
said data representing answers to respective questions automatically acquired from said data sources, in deriving said value for said particular entity.

**10.** A system supporting acquisition and processing of user entered medical information, comprising:

a user interface processor for generating data representing at least one form composition display image enabling a user to compose an electronic form comprising a set of questions to be answered by user data entry via at least one data entry display image; and
a form processor for receiving data entered using a composed electronic form concerning a particular patient and translating received data into a numerical value for use in supporting clinical decision making for said particular patient.

**11.** A system according to claim 10, wherein:

said numerical value is a medical score; and
said user interface processor enables a user to create customized electronic forms comprising score sheets.

**12.** A system according to claim 10, further comprising a database for associating individual questions of said set of questions with one or more data sources for providing data comprising answers to said associated questions.

**13.** A system according to claim 12, further comprising a communication processor for using said database to automatically bidirectionally communicate with said one or more data sources to acquire data representing answers to said associated questions in response to a user command to process a composed form.

**14.** A system according to claim 13, wherein said data sources comprise at least one of: (a) a patient record repository system, (b) a patient administration system, (c) a laboratory system, (d) a pharmacy system, (e) a patient monitoring and/or treatment system.

**15.** A system according to claim 14 wherein said patient monitoring and/or treatment system comprises at least one of: (a) a patient monitor, (b) a ventilation system, (c) an anesthesiology system, and (d) a vital sign acquisition system.

**16.** A system according to claim 12, wherein said database associates said data sources with at least one of: (a) communication protocols and (b) data formats for use in bidirectionally communicating with said data sources to acquire data representing answers to said associated questions.

**17.** A system according to claim 10, wherein said form processor uses
said user data entered in response to said set of questions in said composed form, and
said data representing answers to respective questions automatically acquired from said data sources, in deriving said numerical value for said particular patient.

**18.** A system supporting acquisition and processing of user entered medical information, comprising:

a user interface processor for generating data representing at least one form composition display image enabling a user to compose an electronic form comprising a set of questions to be answered;
a database for associating at least one question of said set of questions with one of a set of data sources for providing data comprising at least one answer to said at least one question of said set of questions; and
a form processor for using:

user data entered using said composed electronic form, and
said data representing answers to associated questions automatically acquired from said data sources using said database,

to derive a numerical value for use in supporting clinical decision making for a particular patient.

**19.** A system supporting acquisition and processing of user entered medical information, comprising:

a user interface processor for generating data representing at least one form composition display image enabling a user to compose an electronic form comprising a set of questions to be answered by user data entry via at least one data entry display image and automatic data acquisition;

a database for associating at least one question of said set of questions with one of a set of data sources for providing data comprising at least one answer to said at least one question of said set of questions;

a communication processor for using said database to automatically bidirectionally communicate with said data sources to acquire data representing answers to associated questions in response to a user command to process said composed form; and

a form processor for using:

user data entered using said composed form, and
said data representing answers to associated questions automatically acquired from said data sources,

to derive a numerical value for use in supporting clinical decision for a particular patient.

20. A method supporting acquisition and processing of user entered information, comprising the activities of:

generating data representing at least one form composition display image enabling a user to compose an electronic form comprising a set of questions to be answered by user data entry via at least one data entry display image; and

receiving data entered using a composed electronic form concerning a particular entity and translating received data into a value for use in supporting decision making for said particular entity.

21. An electronic data storage medium incorporating machine readable instructions for performing the activities of claim 20.

22. A method supporting acquisition and processing of user entered medical information, comprising the activities of:

generating data representing at least one form composition display image enabling a user to compose an electronic form comprising a set of questions to be answered by user data entry via at least one data entry display image; and

receiving data entered using a composed electronic form concerning a particular patient and translating received data into a numerical value for use in supporting clinical decision making for said particular patient.

23. An electronic data storage medium incorporating machine readable instructions for performing the activities of claim 22.

24. A method supporting acquisition and processing of user entered medical information, comprising the activities of:

generating data representing at least one form generation display image enabling a user to compose an electronic form comprising a set of questions to be answered;

associating at least one question of said set of questions with one of a set of data sources providing data comprising at least one answer to said at least one question of said set of questions; and

using,

data entered using a composed electronic form, and
said data representing answers to respective questions automatically acquired from said data sources using said database,

to derive a numerical value for use in supporting clinical decision making for a particular patient.

25. An electronic data storage medium incorporating machine readable instructions for performing the activities of claim 24.

# Fig. 1

# Fig. 2

Fig. 3

**Fig. 4**

**Fig. 5**

Fig. 6